(19) Europäisches Patentamt
European Patent Office
Office européen des brevets

(11) **EP 1 715 429 A1**

(12) **EUROPEAN PATENT APPLICATION**

(43) Date of publication:
**25.10.2006 Bulletin 2006/43**

(51) Int Cl.:
***G06F 17/00*** (2006.01)  ***A61B 5/0285*** (2006.01)

(21) Application number: **06112847.6**

(22) Date of filing: **20.04.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **22.04.2005 JP 2005125760**

(71) Applicant: **FUKUDA DENSHI CO., LTD.**
**Tokyo 113-8483 (JP)**

(72) Inventors:
• **Takahashi, Koji,**
**Fukuda Denshi Co., Ltd.**
**Tokyo (JP)**

• **Nagaya, Motoi,**
**Fukuda Denshi Co., Ltd.**
**Tokyo (JP)**
• **Suzuki, Tsuneo,**
**Fukuda Denshi Co., Ltd.**
**Tokyo (JP)**

(74) Representative: **Lindberg, Olle Nils Olof**
**Albihns Malmö AB**
**P.O. Box 4289**
**203 14 Malmö (SE)**

(54) **Device and method for outputting bioinformation and bioinformation report**

(57) The bioinformation outputting device comprises obtaining unit adapted to obtaining two or more pairs of blood pressure values including the systolic blood pressure value and the diastolic blood pressure value, layout unit adapted to generate a report for presenting the two or more pairs of blood pressure values in a shared two-dimensional region, and outputting unit adapted to output the report. The layout unit lays out, in the two-dimensional region, a plurality of pulse wave patterns each having a pair of the systolic blood pressure value and the diastolic blood pressure value as peak values, so that the report is generated. The report allows intuitive recognition of the relationship with other measured values while facilitating recognition of specific measurement values.

FIG. 3

## Description

FIELD OF THE INVENTION

The present invention relates to an outputting device and method for bioinformation and particularly relates to an outputting device and method for bioinformation serving as an indicator of an arterial disease.

Further, the present invention relates to a bioinformation report for presenting at least bioinformation including blood pressures and particularly relates to a bioinformation report for readily evaluating the possibility of an arterial disease.

BACKGROUND OF THE INVENTION

Conventionally a ratio of blood pressures measured on a lower limb and an upper limb (ankle-brachial blood pressure index) has been generally used as an indicator of a vascular disease such as arteriosclerosis. As an ankle-brachial blood pressure index, for example, a ratio of systolic blood pressures measured on a brachium and an ankle (ABI) and a ratio of systolic blood pressures measured on a brachium and a toe (TBI) have been known. For example, in the case of (lower limb blood pressure/upper limb blood pressure) $\leqq$ 0.9, the indicator is used to indicate the possibility of arteriostenosis on the lower limb.

However, when arteriosclerosis develops through the whole body, an ankle-brachial blood pressure index may present a normal value. Therefore, an ankle-brachial blood pressure index is proposed which is used as a more accurate indicator in combination with a pulse wave propagation velocity or pulse wave velocity (PWV) (for example, see Japanese Patent Laid-Open No. 2000-316821).

However, in order to discriminate an arterial disease, it is necessary to evaluate blood pressure values itself as well as a blood pressure index such as ABI. For example, when a difference in blood pressure value between right and left brachia is 20 mmHg or higher, there is a possibility of hemodynamic disorder on the brachium having a lower blood pressure. In conventional devices for evaluating ABI, the principal object is to present a ratio of blood pressure values. Measured blood pressure values have not been presented using a method for easily knowing respective measured blood pressure values or intuitively knowing the relationship with other measurement values.

SUMMARY OF THE INVENTION

The present invention is devised in view of the problem of the conventional art. An object of the present invention is to provide a bioinformation outputting device and method which can present blood pressure values used as bioinformation serving as an indicator of an arterial disease in such a manner as to allow intuitive recognition of the relationship with other measured values while facilitating recognition of specific measurement values.

That is, a gist of the present invention is a bioinformation outputting device, characterized in that the device comprises obtaining unit adapted to obtain two or more pairs of blood pressure values including the systolic blood pressure value and the diastolic blood pressure value, layout unit adapted to generate a report for presenting the two or more pairs of blood pressure values in a shared two-dimensional region, and outputting unit adapted to output the report, and the layout unit lays out, in the two-dimensional region, a plurality of pulse wave patterns each having a pair of the systolic blood pressure value and the diastolic blood pressure value as peak values, so that the report is generated.

Another gist of the present invention is a method of outputting bioinformation, characterized in that the method comprises: an obtaining step of obtaining two or more pairs of previously measured blood pressure values including the systolic blood pressure value and the diastolic blood pressure value, a layout step of generating report data including the two or more pairs of blood pressure values laid out in a shared two-dimensional region, and an outputting step of outputting the report data, and in the layout step, the report data is generated so as to include a plurality of pulse wave patterns laid out in the two-dimensional region, the pulse wave pattern having a pair of the systolic blood pressure value and the diastolic blood pressure value as peak values.

Still another gist of the present invention is a program for causing a computer to act as the bioinformation outputting device of the present invention or a computer-readable recording medium for storing the program.

According to the present invention, a plurality of pulse wave patterns, each of which has a pair of the systolic blood pressure value and the diastolic blood pressure value as peak values, are laid out in a shared two-dimensional region, so that blood pressure values can be presented in such a manner as to allow intuitive recognition of the relationship with other measurement values while facilitating recognition of specific measurement values.

Other objects and advantages besides those discussed above shall be apparent to those skilled in the art from the description of a preferred embodiment of the invention which follows. In the description, reference is made to accompanying drawings, which form a part thereof, and which illustrate an example of the various embodiments of the invention. Such example, however, is not exhaustive of the various embodiments of the invention, and therefore reference is made to the claims which follow the description for determining the scope of the invention.

BRIEF DESCRIPTION OF THE DRAWINGS

The accompanying drawings, which are incorporated in and constitute a part of the specification, illustrate embodiments of the invention and, together with the description, serve to explain the principles of the invention.

FIG. 1 is a block diagram showing a structural example of a bioinformation measuring device which is an example of a bioinformation outputting device according to an embodiment of the present invention.

FIG. 2 is a diagram showing an example of formulas for calculating a blood vessel elasticity in the bioinformation outputting device according to the embodiment.

FIG. 3 is a diagram for explaining an example of the format of a report outputted by the bioinformation outputting device of First Embodiment.

FIG. 4 is a diagram for explaining an example of another format of a report outputted by the bioinformation outputting device of First Embodiment.

FIG. 5 is a diagram showing an example where measurement values indicating a possibility of slight artery occlusion are represented in the report format of FIG. 3.

FIG. 6 is a diagram showing an example where measurement values indicating a possibility of serious artery occlusion are represented in the report format of FIG. 3.

FIGs. 7A and 7B are diagrams for explaining an example of the format of a report outputted by a bioinformation outputting device according to Second Embodiment.

FIG. 8 is a diagram showing an example where the blood pressure measurement value report of the embodiment is applied to a total report including other measurement results.

FIG. 9 is a flowchart for explaining the output of the report in the bioinformation outputting device according to the embodiment of the present invention.

DETAILED DESCRIPTION OF THE PREFERRED EMBODIMENTS

Preferred embodiments of the present invention will be specifically described below in accordance with the accompanying drawings.

(First Embodiment)

(Device Configuration)

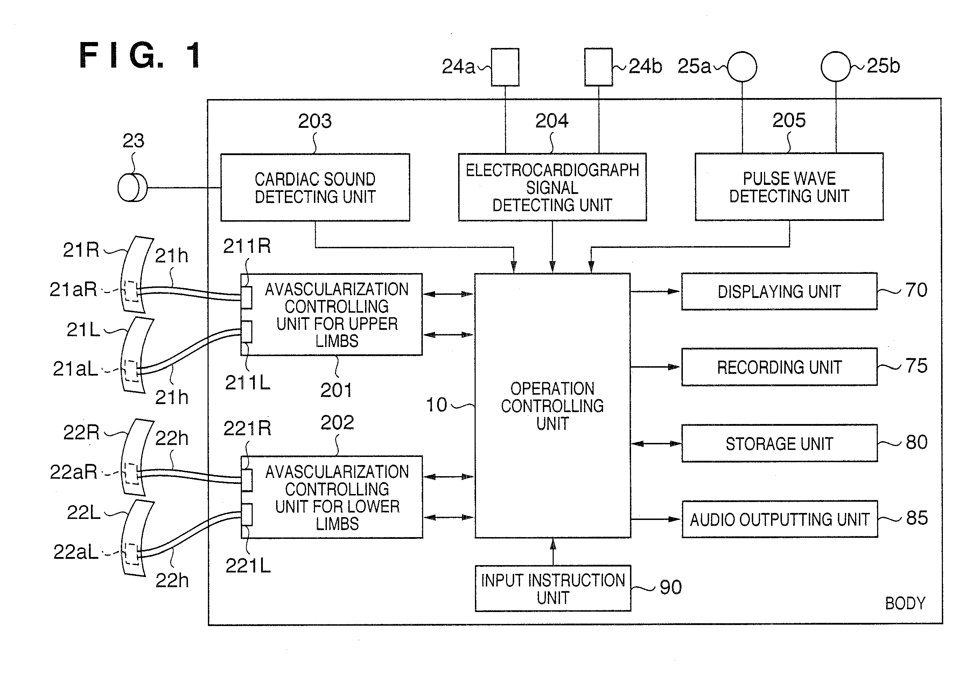

FIG. 1 is a block diagram showing a structural example of a bioinformation measuring device which is an example of a bioinformation outputting device according to an embodiment of the present invention.

The present embodiment will describe an example in which the present invention is applied to a bioinformation measuring device capable of obtaining kinds of bioinformation. In the bioinformation outputting device of the present invention, at least the systolic blood pressure value and the diastolic blood pressure value have to be obtained in some way. It is further preferable to obtain pulse wave measurement results at a site where blood pressures are measured or around the site. The measurement results may be obtained by, for example, reading data from a storage device or the like. In this case, the function of measuring blood pressure values and pulse waves is not necessary.

An operation controlling unit 10 controls the operations of the overall bioinformation measuring device of the present embodiment. The operation controlling unit 10 is, for example, a general-purpose computer including a CPU, ROM, RAM (including nonvolatile RAM) and various interfaces (not shown). For example, the CPU executes control programs stored in ROM and bulk memory such as an internal or external hard disk and an optical disk, so that the operations discussed below are performed and controlled. As a matter of course, all the operation are not necessarily processed by software and at least some of the operations may be implemented by hardware.

The operation controlling unit 10 controls an avascularization controlling unit 201 for upper limb and an avascularization controlling unit 202 for lower limb to measure a diastolic blood pressure Pd, a systolic blood pressure Ps, and a mean blood pressure Pm on an upper limb (e.g., a brachium) and a lower limb (e.g., an ankle or toe). ABI (the ratio of a blood pressure value on a brachium (representative) and a systolic blood pressure value on a right or left ankle) and TBI (the ratio of a blood pressure value on a brachium (representative) and a systolic blood pressure on a right or left toe) may be determined from the measurement results.

The operation controlling unit 10 can further calculate a pulse wave propagation velocity between the heart (aortic valvular opening) and an ankle, between the heart and a toe, or between sites where cuffs are attached, by using pulse wave signals supplied from the avascularization controlling unit 201 for upper limb and the avascularization controlling unit 202 for lower limb (a cardiac sound signal supplied from a cardiac sound detecting unit 203, an electrocardiograph signal supplied from an electrocardiograph signal detecting unit 204, and a carotid pulse, a femoral artery pulse, and a popliteal artery pulse or the like supplied from a pulse wave detecting unit 205 may be selectively used when necessary) and a blood vessel length

(corresponding to a predetermined blood vessel length) between measured sites.

In response to the control of the operation controlling unit 10, the avascularization controlling unit 201 for upper limb and the avascularization controlling unit 202 for lower limb control the pressurization/depressurization (avascularization) of the compression bladders (21aR, 21aL, 22aR, 22aL) of cuffs 21R and 21L and 22R and 22L, which are connected via hoses 21h and 22h, by using pumps, exhaust valves, and so on (not shown). The avascularization controlling unit 201 for upper limb and the avascularization controlling unit 202 for lower limb comprise sensors for detecting pulse waves propagating through the hoses 21h and 22h, for example, pressure sensors (211R and L and 221R and L) are provided by which pulse waves propagating through the compression bladders and hoses are converted to electric signals and outputted to the operation controlling unit 10. In FIG. 1, the avascularization controlling unit 201 for upper limb and the avascularization controlling unit 202 for lower limb are separated from each other but may be provided as one unit. In the following explanation, cuffs 21 and cuffs 22 not including R or L indicate both of right and left cuffs 21 and 22.

The cardiac sound detecting unit 203 supplies, to the operation controlling unit 10, a cardiac sound signal having been detected from a subject by a cardiac sound microphone 23. The cardiac sound signal is mainly used for determining the starting point of a pulse wave in the heart.

The electrocardiograph signal detecting unit 204 obtains electrocardiograph signals detected from electrocardiograph electrodes 24a and 24b and supplies the signals to the operation controlling unit 10. The electrocardiograph signals are obtained as necessary in more comprehensive diagnosis.

The pulse wave detecting unit 205 supplies, to the operation controlling unit 10, pulse waves having been detected by the pulse wave sensors 25a and 25b, to be specific, a carotid pulse, a femoral artery pulse, and a popliteal artery pulse. Moreover, sensors similar to pulse wave sensors 25a and 25b may be disposed on sites where the cuffs 21 and 22 are attached (including sensors in the cuffs 21 and 22) and pulse waves may be detected from these pulse wave sensors.

Further, the operation controlling unit 10 is connected to a displaying unit 70 which can display/output various kinds of operation guidance, measurement results and diagnosis indicators, a recording unit 75 which is an image forming device such as a printer capable of recording and outputting measurement results and diagnosis indicators, a saving unit 80 including, for example, a hard disk drive, a writable optical disk drive, and nonvolatile semiconductor memory for storing measurement results and diagnosis indicators, an audio outputting unit 85 which can output sound of guidance and kinds of alarm sounds, and an inputting/command unit 90 which includes a keyboard, a mouse, a button, and a touch panel and enables input and instruction for the user.

Additionally, the following may be provided: a wire and/or radio communications interface for communications with other devices, and a storage device or the like using a removable medium. The displaying unit 70 and the recording unit 75 may be connected outside. In other words, in addition to the displaying unit 70 and the recording unit 75 which are included in the body of the device, an external display device with a larger display area and/or a larger number of display colors and an external recording device with a larger printing area and/or a larger number of printed colors may be connected. With this configuration, it is possible to simultaneously obtain a smaller body and a variety of outputs. In this case, a known display interface or printer interface is provided.

(Measurement: Preparation before Measurement)

The following will describe steps and operations for measurement using the bioinformation outputting device configured thus. Measurement with the highest accuracy will be discussed below. Initialization for the operations of the device, for example, time setting has been completed.

First, for preparation, the cuffs, sensors and so on are attached to a subject. To be specific, the cuffs 21R/21L for upper limb are attached to the right/left brachia of the subject and the cuffs 22R/22L for lower limb are attached to the right/left ankles or toes of the subject. Although the cuffs attached to ankles and toes are different in configuration, the cuffs will be both described as cuffs 22 for lower limb. The cuffs 21 and 22 can be attached by a hook and loop fastener or the like. Further, the electrocardiograph electrodes 24a and 24b are attached to, for example, left and right wrists. For preferable detection, cream or the like is applied to the attachment sites as usual. The sites where the electrocardiograph electrodes are attached can be changed according to the kind of obtained lead. In the case where pulse waves are measured by a mechanism other than the cuffs, for example, separate pulse wave sensors on the sites where blood pressures are measured or around the sites, the pulse wave sensors are attached at this point of time.

The cardiac sound microphone 23 is attached with a tape to a predetermined position on the chest of the subject (II interspace sternum edge). Moreover, the pulse wave sensor 25a is attached to the carotid artery pulsation part of the neck. Moreover, the femoral artery pulse sensor 25b is attached to the groin when necessary.

Subsequently, subject's personal information including age, sex, height and weight is inputted using the inputting/command unit 90. According to a predetermined formula based on the value of the height, the corresponding blood vessel length is determined. In some cases, the blood vessel length is determined according to a predetermined formula by measuring distances between the sites where the cuffs 21 and 22 are attached and the II interspace sternum edge with a scale or the like, and inputting the distances. Preparation before measurement is completed thus.

(Measurement: Pulse Wave Measurement)

When preparation for measurement is completed and an instruction to start measurement is issued from, for example, the inputting/command unit 90, the operation controlling unit 10 first starts measuring pulse waves on sites where blood pressures are measured or around the sites. When pulse waves are measured using the cuffs, the order of measurement can be arbitrarily set. First, an instruction to start pressurization to the right brachial cuff 21R is issued to the avascularization controlling unit 201 for upper limb.

The avascularization controlling unit 201 for upper limb injects air into the cuff 21R to inflate the compression bladder 21aR. At this point, the pressure applied by the cuff 21R to a body surface (cuff internal pressure detected by the pressure sensor 211R) can be set low as long as a pulse wave can be detected. For example, the pressure can be lower than the typical diastolic blood pressure value.

A pulse wave propagates as a pressure wave of air from the compression bladder 21aR through the hose 21h and is detected by the pressure sensor 211R. The pulse wave is converted to an electric signal (generally a pressure sensor converts a pressure into an electric signal and outputs the signal) and outputted to the operation controlling unit 10 as a pulse wave signal obtained from the cuff 21R. The operation controlling unit 10 records the pulse wave signal in the saving unit 80. When a predetermined amount of pulse waves is measured with a sufficient amplitude, the exhaust valve is opened by the avascularization controlling unit 201 for upper limb to evacuate the cuff 21R.

These pulse wave measurement operations are performed simultaneously or sequentially on all the cuffs and pulse waves are measured on sites where blood pressures are measured or around the sites. When pulse waves are detected by the pulse wave sensors different from those of the cuffs, pulse waves may be obtained simultaneously or sequentially from all the sensors and recorded.

(Measurement: Blood Pressure Measurement)

Subsequently the operation controlling unit 10 starts measuring blood pressures. Also in this case, the order of measurement can be arbitrarily set. First an instruction to start pressuring the right brachial cuff 21R is issued to the avascularization controlling unit 201 for upper limb.

The avascularization controlling unit 201 for upper limb injects air into the cuff 21R, inflates the compression bladder 21aR, and pressurizes an attachment site. A pulse wave propagates as a pressure wave of air from the compression bladder 21aR through the hose 21h in response to the pressurization, and the pulse wave is detected by the pressure sensor 211R. The pulse wave is converted to an electric signal (generally a pressure sensor converts a pressure into an electric signal and outputs the signal) and outputted to the operation controlling unit 10 as a pulse wave signal obtained from the cuff 21R.

The operation controlling unit 10 causes the avascularization controlling unit 201 for upper limb to inject air into the compression bladder 21aR until a pulse wave detected by the pressure sensor 211R becomes too small to be detected as a waveform and the attachment site of the cuff is avascularized or until the pressure reaches a predetermined cuff pressure at which the attachment site of the cuff is sufficiently avascularized. And then, pressurization is stopped when a pulse wave becomes too small to be detected as a waveform. The cuff pressure (internal pressure) at this point can be detected by the pressure sensor 211R. And then, the avascularization controlling unit 201 for upper limb is instructed to gradually reduce the cuff pressure.

The avascularization controlling unit 201 for upper limb adjusts the exhaust valve (not shown) and starts recording detected pulse wave signals while depressurizing the cuff at a constant rate by releasing air from the compression bladder 21aR. Cuff pressures are also continuously detected by the pressure sensor 211R. The detection of pulse waves is started again in the process of depressurization, and a systolic blood pressure Ps, a mean blood pressure Pm, and a diastolic blood pressure Pd are determined by cuff pressures on a point where a pulse wave rapidly increases in amplitude, a point where a pulse wave has the maximum amplitude, and a point where a pulse wave rapidly decreases in amplitude. The cuff pressure can be calculated also by a value at the start of depressurization, a depression rate, and a decompression time. Such a method of measuring blood pressures is known as oscillometric method. When the diastolic blood pressure is determined, the cuff is depressurized without stopping.

Such a process for blood pressure measurement is similarly performed on the other cuffs 21L, 22R and 22L, and the blood pressure measurement of upper and lower limbs is completed.

The measured diastolic blood pressure value, systolic blood pressure value, and mean blood pressure value are associated with the pulse wave signals having been detected at the same site and the values are stored in the saving unit 80.

(Measurement: PWV measurement)

With the above measurement results of blood pressures and pulse waves, a report (described later) can be generated. However, in the present embodiment, another bioinformation is measured to obtain another useful indicator for the diagnosis of an arterial disease. The following will first discuss the measurement of PWV. When carotid pulses are detected using the pulse wave sensor 25a, the operation controlling unit 10 obtains the pulse waves through the pulse wave detecting unit 205 and detects the generation of cardiac sound (e.g., II sound) corresponding to the rising edge of the pulse wave from a cardiac sound signal having been obtained through the cardiac sound detecting unit 203. The pulse waves and the cardiac sound signal undergo proper processing such as AD conversion and are stored in the saving unit 80. PWV is determined as follows:

$$PWV = AF/(t + tc)$$

where AF represents a blood vessel length to the II interspace sternum edge and the site (ankle or toe) where the cuff 22R is attached, t represents a time difference from the rising edge of a carotid pulse (or brachial pulse wave) to the rising edge of an ankle (or toe) pulse wave, and tc represents a time difference from the rising edge of the II sound of cardiac sound to the notch point of the carotid pulse (or brachial pulse wave).

When the pulse wave sensor 25a is not used, PWV (also referred to as baPWV) may be determined as follows: an estimated blood vessel length is determined by a difference between a distance from the heart (the starting point of an aorta) to the site where the cuff 21R is attached and a distance from the heart (the starting point of an aorta) to the site where the cuff 22R is attached, and the estimated length is divided by a time difference between the rising point of a pulse wave measured by the cuff 21R attached to a brachium and the rising point of a pulse wave detected by the cuff 22R. Alternatively PWV may be similarly determined by a time difference between pulse waves measured on two given points and a blood vessel length (or its reduced value) between the points of measurement.

At the completion of measurement of PWV, the operation controlling unit 10 causes the avascularization controlling unit 201 for upper limb and the avascularization controlling unit 202 for lower limb to release the cuffs, stores the measurement results in the saving unit 80, and completes the process of measurement.

As described above, blood pressures and pulse waves are measured on the right and left brachia and right and left ankles/toes in the present embodiment. In order to attain the effect of a report format (described later) which is a characteristic of the present embodiment, blood pressures and pulse waves are measured on at least two points of a living body. Further, PWV may be calculated between the heart and ankle and between the heart and toe, and may be calculated both on the right and left ankles or toes. Moreover, PWV may be calculated between the heart (the starting point of an aorta) and brachium, between the heart and knee, between the thigh and ankle, and between the brachium and wrist.

(Calculation of blood vessel elasticity)

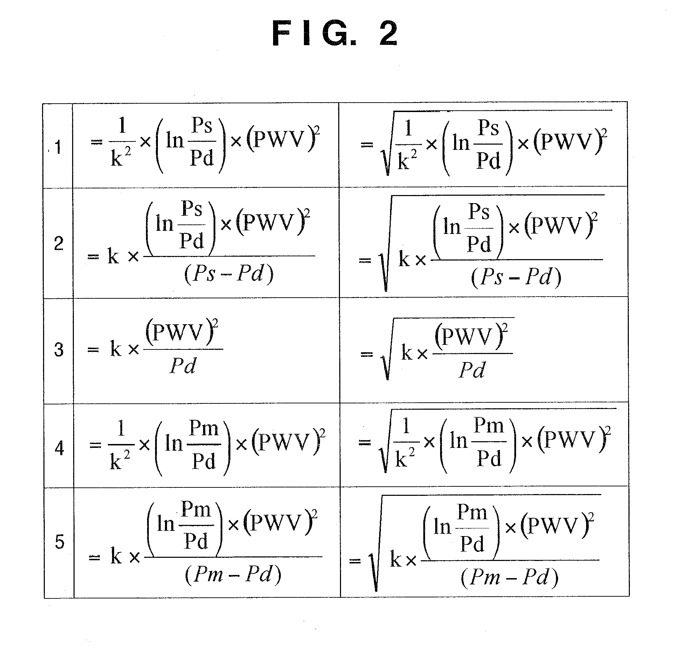

Subsequently the operation controlling unit 10 calculates blood vessel elasticity. The blood vessel elasticity may be values determined by, for example, Formulas 1 to 5 shown in FIG. 2 where k represents a constant. Which formula should be used is determined beforehand. As a matter of course, the blood vessel elasticity can be calculated using two or more of the formulas.

Blood pressure values (systolic blood pressure Ps, mean blood pressure Pm, and diastolic blood pressure Pd) used in Formulas 1 to 5 are ideally blood pressure values at the center points of sections where PWVs have been measured. For example, measurement values on a brachium can be used in place of the blood pressure values. The blood pressure value at the center point may be estimated based on a predetermined conversion formula and a measurement value of blood pressure at a site where pulse waves have been measured to calculate PWV. Further, PWV used for calculating the blood vessel elasticity may be any one of measurement results obtained between the heart and ankle, between the heart and toe (or between other two points).

Moreover, it is preferable to store the measured bioinformation and values such as calculated PWVs and blood vessel elasticity so as to correspond to the personal information of the subject and the date and time of measurement. For example, a folder or directory is generated for each subject, a folder or directory is further generated in the folder or directory of the subject for each time of measurement, and then obtained blood pressure values and pulse wave signals or the like are stored in the folder or directory.

As mentioned at the beginning of the present embodiment, the present embodiment described an example where the present invention is applied to the bioinformation measuring device capable of obtaining kinds of bioinformation. Hence, the measurements of other kinds of bioinformation, to be specific, the measurements of pulse waves, electrocardiograms, cardiac sounds or the like were described and the calculation of PWV and blood vessel elasticity was described in addition to the measurements of pulse waves and blood pressures. The minimum requirement of the bioinformation outputting device of the present invention is the function of outputting a report in the following format based on the measurement results of pulse waves and blood pressure values.

For example, in the simplest configuration of the bioinformation outputting device capable of outputting a report in the format below, software for outputting the report discussed below is installed in a computer which is commercially available as a personal computer. In this case, the measurement results of bioinformation including blood pressures and pulse waves can be obtained from a removable medium or a network. Also in this case, the report may be outputted to either an internal or external display of the computer and a printer directly or indirectly connected to the computer.

(Output of Report)

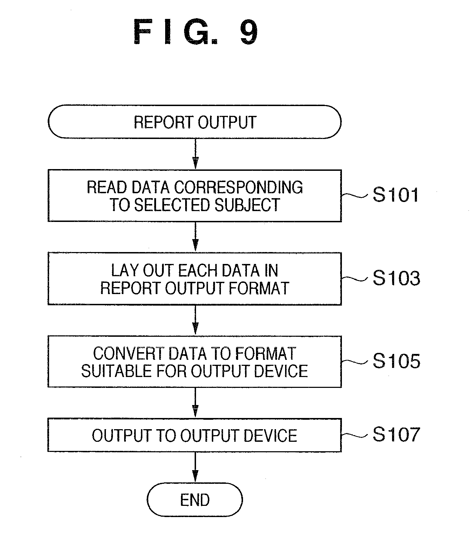

Referring to the flowchart of FIG. 9, the output of a report in the bioinformation outputting device will be described below. In this case, the report is outputted by, for example, the recording unit 75 which is a color printer. As described above, the report may be outputted to an external display device or an external outputting device as well as the displaying unit 70.

The report may be automatically outputted after measurement. For example, a list of past subjects in the saving unit 80 can be displayed by the displaying unit 70 and measurement results can be outputted regarding a subject having been selected from the list by the inputting/command unit 90. In this case, measurement results are outputted regarding a subject having been selected from the list of past subjects.

First, the operation controlling unit 10 reads the personal information and measurement results of the selected subject from the saving unit 80 (step S101). Then, the read information is converted to a predetermined display format and laid out in an output format (described later) (step S103). In this step of the present embodiment, each representative pulse wave is deformed (scaled) described later, the mean blood pressure is plotted, and the measurement results of each site are laid out.

At the completion of the layout, the information is converted to a format for enabling output in the destination of output, for example, the recording unit 75 (step S105). The format conversion includes, for example, resolution conversion corresponding to the destination of output (72 or 96 dpi for display and 400 to 600 dpi for printing), color conversion (conversion to monochrome output, increase/reduction in the number of colors, and so on), scaling, and conversion to a bitmap. After the conversion to a format suitable for the destination of output, the converted data is outputted to the destination as report data (step S107) and then displayed or printed.

As long as the bioinformation outputting device can obtain previously measured data through a network or a removable storage medium or the like, even if the bioinformation outputting device does not have the function of measuring bioinformation, a report can be outputted by similar processing. In other words, the bioinformation outputting device first obtains list information on measurement data (including a list of subjects' names) and displays the information on a display. After a subject is selected using an inputting device such as a keyboard and a mouse, processing is performed as in steps S101 to S105, and the information is outputted to a predetermined destination of output, for example, a display or a printer (step S107). Thus, a similar report is displayed or printed.

(Format of Bioinformation Report)

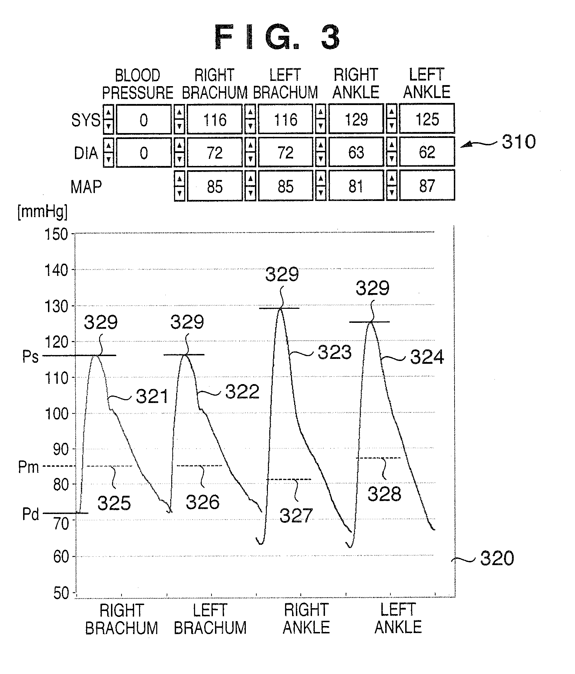

FIG. 3 shows an example of a bioinformation report outputted by the bioinformation outputting device of the present embodiment. The following will only discuss the report format of blood pressure measurement values, which is a characteristic of the present embodiment. It is needless to say that the report may include at least one or more of the other measurement values.

The report includes a two-dimensional region 320 having orthogonal axis. The vertical axis represents blood pressure values. The present embodiment is characterized by visually presenting the systolic and diastolic blood pressure values and a difference between the values with pulse wave patterns. Another characteristic is to present an indicator (mean blood pressure indicator) for indicating the mean blood pressure in addition to the waveform, in order to easily know the relationship of the mean blood pressure value at each measurement site.

In the example of FIG. 3, four pulse wave patterns (waveforms of four pulse waves for a beat) 321 to 324 are arranged as a list in a lateral direction relative to the scale of the vertical axis (scaled when necessary) such that the maximum peak and the minimum peak indicate the systolic (maximum) blood pressure value Ps and the diastolic (minimum) blood pressure value Pd which are measured at the same site. Although the adjacent pulse wave patterns may overlap each other, a smaller overlap is more preferable in consideration of higher visibility of the respective waveforms.

A specific blood pressure value can be easily read based on the peak position of the pulse wave pattern and the amplitude (height) of the pulse wave pattern is known, so that a difference between the systolic blood pressure and the diastolic blood pressure can be known at a glance. It is also possible to add horizontal supplementary lines 329 each indicating the peak position equivalent to the systolic blood pressure. When the horizontal supplementary lines 329 are added, the relationship of the systolic blood pressure values can be more clearly known. The length of the horizontal supplementary line 329 is not particularly limited but in order to readily compare the systolic blood pressure value with that of another site, it is preferable that the ends of the horizontal supplementary lines at the systolic blood pressure values of two sites vertically overlap. The two sites are at least a combination of upper right and left limbs, lower right and left limbs, upper and lower right limbs, and upper and lower left limbs.

Further, measurement values at each site (or at a different time at the same site) are indicated in the same region, so that the respective measurement values can be confirmed at a time and a comparison can be easily and properly performed on measurement values obtained at another site (or another date and time, at a different site and/or time though not particularly mentioned below) or on a difference (pulse pressure) between the systolic blood pressure and the diastolic blood pressure.

Further, in the present embodiment, indicators 325 to 328 for indicating mean blood pressure values are presented in addition to the pulse wave patterns. It is thus possible to know a mean blood pressure value not presented by a peak value and easily know correlation with the measurement values of other sites.

In the example of FIG. 3, the indicators 325 to 328 for indicating the mean blood pressure values are presented as horizontal dotted lines in the waveforms. As long as correspondence with the pulse wave patterns (points of measurement) can be clearly known and actual measured values can be identified, the layout and shapes of the indicators 325 to 328 are not limited.

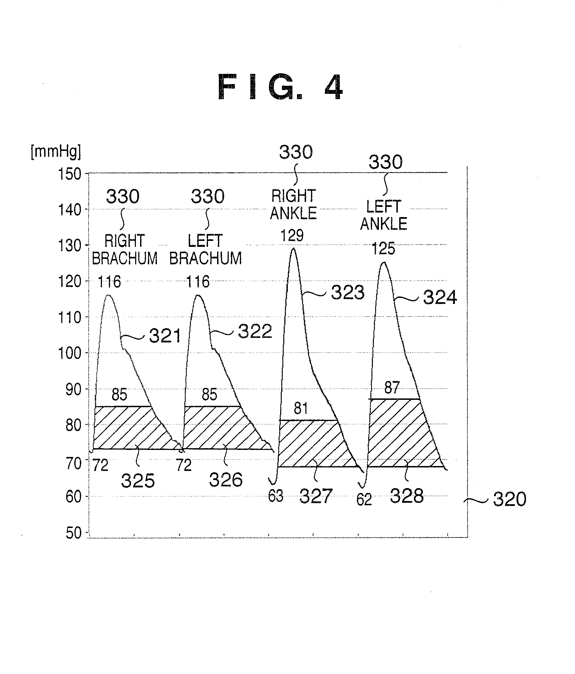

For example, the same dotted line may be partially or entirely disposed outside the waveform, and marks like • and ■ may be used in addition to the indicators of dotted lines each having a certain length. For example, as shown in FIG. 4, the indicator for indicating the mean blood pressure may be an indicator visually indicating a region corresponding to blood pressure values equal to or lower than the mean blood pressure value of each waveform. For example, an indicator which fills the region with a hatched pattern or solid fills the region may be used. Needless to say, the indicator may be an indicator visually indicating a region corresponding to blood pressure values equal to or higher than the mean blood pressure value.

Further, the pulse wave patterns can be made visually different for the respective measurement sites. To be specific, pulse wave patterns and mean blood pressure indicators can be presented using different colors and patterns for the respective measurement sites. As shown in the example of FIG. 4, the sites where the waveforms (and the mean blood pressure indicators) are measured may be clearly indicated by characters 330 in the report.

The actual measured values of blood pressures may be specified. For example, the values may be specified in a table like a table 310 of FIG. 3. Alternatively as shown in FIG. 4, a specific numeric value may be presented on at least one of the peak position of the pulse wave pattern and a point around the mean blood pressure indicator.

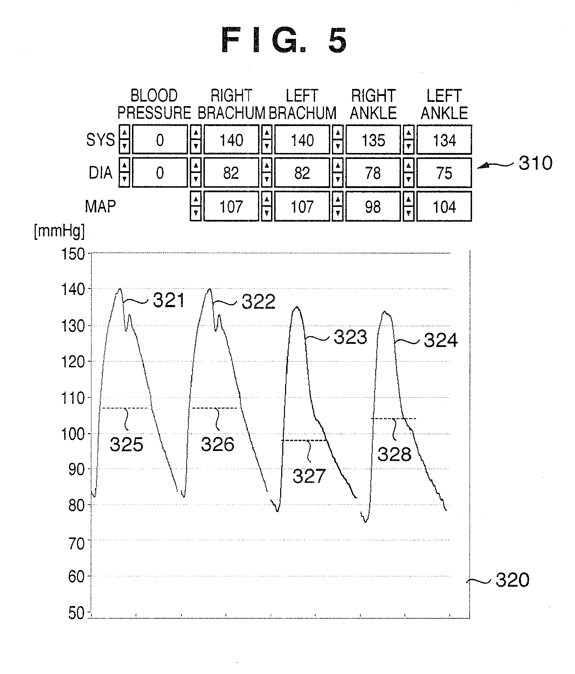

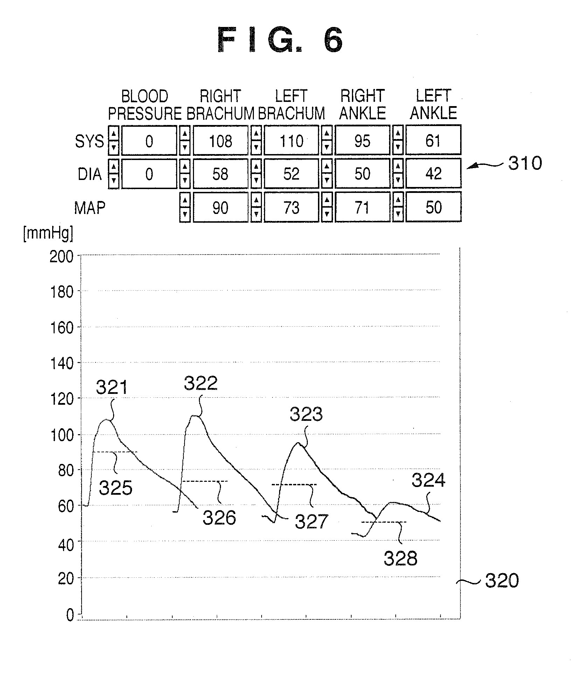

FIGs. 5 and 6 show, in the format of FIG. 3, the measurement results indicating suspicions of slight artery occlusion and serious artery occlusion, respectively.

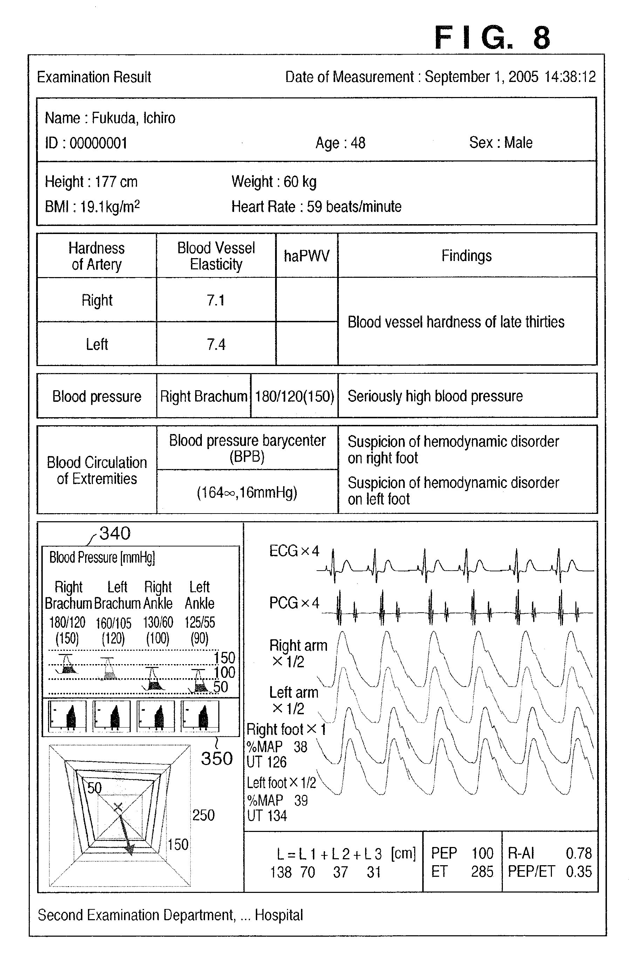

FIG. 8 shows an example where the report of blood pressure measurement values of the present embodiment is applied to a total report including another measurement results. In FIG. 8, the report of blood pressure measurement results is presented in a region denoted as 340. In the example of FIG. 8, a color is changed for each measurement site and a mean blood pressure indicator is used which solid fills a region not higher than the mean blood pressure value in a waveform as shown in FIG. 4. The measurement sites and actual measured values are presented as characters.

In the example of FIG. 8, a pulse wave amplification graph 350 is presented under the region 340 as information for confirming the credibility of blood pressure measurement performed using the cuffs. The pulse wave amplitude graph 350 includes a list of the width values of pulse waves which are detected by the cuffs when blood pressures are measured by oscillometric method. In other words, the horizontal axis represents a cuff pressure (decreasing to the right) and the vertical axis represents a pulse wave amplitude value. The quality of blood pressure measurement can be mainly known based on the shape of the graph, and the extension of a blood vessel can be also known. Such a graph can be generated using pulse wave signals and cuff pressures detected during blood pressure measurement.

With the expression using numeric values and pulse wave patterns and a list of the pulse wave amplitude graphs, it is possible to comprehensively check occlusion as well as the systolic blood pressure. By referring to pulse wave amplitude graphs in addition to the display of pulse wave patterns, it is possible to more accurately evaluate whether a blood pressure difference between upper and lower limbs or between right and left limbs is an error of blood pressure measurement or a significant difference.

In consideration of the provision of information useful for diagnosis, it is particularly preferable that pulse wave patterns for presenting blood pressure measurement values are measured at a site where blood pressure values are measured or on a point around the site. In this case, of pulse waves measured before blood pressure measurement, the representative waveform of one beat (for example, a waveform having the maximum amplitude) can be selected and used.

The pulse wave pattern used for the report can be generated by scaling, in the width direction (time axis direction), the original waveform according to the size (width) of a layout region in a predetermined two-dimensional region, and scaling, in the height direction (amplitude direction), the original waveform according to the systolic blood pressure value and diastolic blood pressure value to be presented as peak values and according to the scale of the axis of blood pressure values.

In the present embodiment, a pulse wave measured before blood pressure measurement is used. The pulse wave may be measured after a lapse of a predetermined time since blood pressure measurement. When a blood pressure value is measured using a pulse wave as in the present embodiment, a pulse wave detected for blood pressure measurement may be used.

A specific blood pressure value can be easily known by presenting the blood pressure value with a pulse wave pattern, and the relationship with another blood pressure measurement value can be intuitively known. This effect can be attained without using an actually measured pulse wave pattern. Thus, a prepared model waveform can be properly scaled and used for a report.

As described above, according to the present embodiment, the systolic and diastolic blood pressure values are presented by the peaks of a pulse wave pattern, so that the specific measurement values of the respective sites can be easily known and the relationship with another measurement value can be intuitively known. Therefore, the indicator of the present embodiment is quite useful for deciding whether there is a possibility of artery occlusion and deciding the site and level of occlusion. Further, the indicator for indicating the mean blood pressure value is presented in addition to the pulse wave pattern, so that the mean blood pressure value can be known and compared with ease.

Moreover, the pulse wave pattern used for indicating blood pressure values is a pulse wave pattern actually measured at a site where blood pressure values are measured or on a point around the site, so that information can be obtained also from the waveform itself and a more useful report can be achieved.

(Second Embodiment)

The following will describe a bioinformation outputting device according to Second Embodiment of the present invention. Since Second Embodiment is similar to First Embodiment except for the format of an outputted report, only the output format of a report, which is a characteristic of the present embodiment, will be described below.

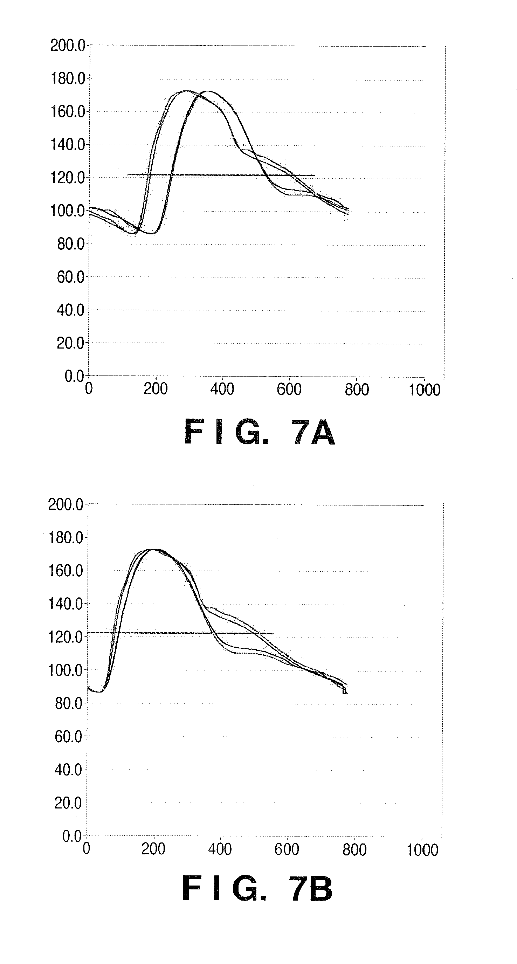

FIGs. 7A and 7B are showing the format of a report outputted by the bioinformation outputting device of the present embodiment. The present embodiment is similar to First Embodiment in that respective blood pressure measurement values are represented as pulse wave patterns, and the present embodiment is characterized by a plurality of superimposed pulse wave patterns. Preferably the pulse wave patterns are superimposed by aligning its time axes such that a difference of the waveforms can be readily known. In other words, the plurality of pulse wave patterns are preferably presented in synchronization with one another. Such synchronization can be obtained by detecting, for example, the rising points of the pulse wave patterns and laying out the pulse wave patterns so as to dispose the rising points on the same position in the horizontal axis direction.

This expressing method enables a detailed comparison between sites where similar pulse waves are measured, for example, a detailed comparison between a right brachium and a left brachium. Further, a time UT (Upstroke Time) from the rising point to the peak of a waveform tends to increase in the presence of artery stenosis, and thus the times UT can be intuitively compared with each other by the pulse wave patterns superimposed in synchronization with each other. In this point, this method is quite effective. In the example of FIG. 7A, measurement values on an upper limb are superimposed and measurement values on a lower limb are superimposed. As shown in FIG. 7B, measurement results on right and left brachia and right and left ankles can be all superimposed.

In the present embodiment, for at least mean blood pressure indicators corresponding to the plurality of superimposed pulse wave patterns, it is preferable to use visually different expressing methods (color, patterns, and so on). Also in the present embodiment, characters for specifying specific measurement values or measurement sites can be added as in First Embodiment. However, an expression using filling shown in FIG. 4 is not preferable for the mean blood pressure indicator.

As described above, according to the bioinformation outputting device of the present embodiment, the plurality of pulse wave patterns are superimposed, so that a difference between the pulse wave patterns can be more clearly known in addition to the effect of First Embodiment.

(Another Embodiment)

The foregoing embodiments only described the case where blood pressures and pulse waves are measured on two points of an upper limb and two points of a lower limb and all the measurement results are collectively presented in a graph. The essence of the present invention is, however, to present at least two measurement results of blood pressures so as to enable a comparison by using pulse wave patterns. Needless to say, the present invention is also applicable to a report indicating results measured at the same site and different times.

Moreover, the method of measuring blood pressures and pulse waves is not limited. Blood pressure values may be measured by a method other than oscillometric method. For example, a method using Korotkov sound or ultrasound, a method using light, or an invasive method may be used to measure blood pressures. Pulse waves can be measured by any given method as long as the pulse waves are measured at a site where blood pressures are measured or on a point around the site.

Further, the format of the report and measurement results included in the report can be selected by the user, and the scale of the graph can be changed by the user.

The output of the report of the foregoing embodiments can be performed also by, for example, a computer which reads previously measured blood pressure values and pulse wave signals from a storage device for storing the values and signals, and outputs the report in the above format. Therefore, it is needless to say that the present invention comprises a program for causing the computer to perform the output of the report of the foregoing embodiments or a computer-readable recording medium for storing the program.

As many apparently widely different embodiments of the present invention can be made without departing from the spirit and scope thereof, it is to be understood that the invention is not limited to the specific embodiments thereof except as defined in the appended claims.

## Claims

1. A bioinformation outputting device, **characterized by** comprising:

   obtaining unit adapted to obtain two or more pairs of blood pressure values including a systolic blood pressure value and a diastolic blood pressure value;
   layout unit adapted to generate a report for presenting the two or more pairs of blood pressure values in a shared two-dimensional region; and
   outputting unit adapted to output the report, wherein the layout unit lays out, in the two-dimensional region, a plurality of pulse wave patterns each having a pair of the systolic blood pressure value and the diastolic blood pressure value as peak values, so that the report is generated.

2. The bioinformation outputting device according to claim 1, **characterized in that** the blood pressure values further include a mean blood pressure value, and
   the layout unit lays out an indicator for indicating the mean blood pressure value, the indicator being associated with the pulse wave pattern having the corresponding systolic blood pressure value and diastolic blood pressure value as peak values.

3. The bioinformation outputting device according to claim 2, **characterized in that** the indicator for indicating the mean blood pressure value is a linear indicator.

4. The bioinformation outputting device according to claim 2, **characterized in that** the indicator for indicating the mean blood pressure value is an indicator for visually indicating a region corresponding to blood pressure values equal to or lower than the mean blood pressure value of the pulse wave pattern or blood pressure values equal to or higher than the mean blood pressure value.

5. The bioinformation outputting device according to claim 1, **characterized in that** the pulse wave pattern is a pulse wave pattern measured almost at the same site as measurement of the blood pressure values.

6. The bioinformation outputting device according to claim 1, **characterized in that** the pulse wave pattern is a pulse wave pattern measured almost at the same site as measurement of the blood pressure values, and
   the layout unit lays out the plurality of pulse wave patterns in synchronization one another in the two-dimensional region.

7. The bioinformation outputting device according to any one of claims 1 to 6, **characterized in that** the blood pressure values include a blood pressure value measured on an upper limb and a lower limb.

8. The bioinformation outputting device according to any one of claims 1 to 6, **characterized in that** the layout unit lays out the pulse wave patterns such that the pulse wave patterns are visually different from one another.

9. The bioinformation outputting device according to any one of claims 1 to 6, **characterized in that** the obtaining unit comprises:

   pulse wave detecting unit adapted to detect a pulse wave by pressurizing a body surface of a living body with a cuff; and
   measuring unit adapted to measure the blood pressure values based on the pulse wave and a pressurizing force of the cuff.

10. The bioinformation outputting device according to any one of claims 1 to 6, **characterized in that** the outputting unit is a display device, an image forming device, or a communication device.

11. A method of outputting bioinformation, **characterized in that** the method comprises:

    an obtaining step of obtaining two or more pairs of previously measured blood pressure values including a systolic blood pressure value and a diastolic blood pressure value;
    a layout step of generating report data for presenting the two or more pairs of blood pressure values in a shared two-dimensional region; and
    an outputting step of outputting the report data, wherein in the layout step, the report data is generated so as to include a plurality of pulse wave patterns laid out in the two-dimensional region, the pulse wave pattern having a pair of the systolic blood pressure value and the diastolic blood pressure value as peak values.

12. A bioinformation report for presenting bioinformation including at least a systolic blood pressure value and a diastolic blood pressure value,
    **characterized in that** the report has a blood pressure measurement result output region including pulse wave patterns laid out in a two-dimensional region specified by an orthogonal coordinate system having a vertical axis representing a blood pressure value, the pulse wave pattern having the systolic blood pressure value and the diastolic blood pressure value as peak values.

13. The bioinformation report according to claim 12, **characterized in that** measurement results of the plurality of systolic blood pressure values and diastolic blood pressure values are indicated by the plurality of pulse wave patterns laid out in the two-dimensional region.

14. The bioinformation report according to claim 12, **characterized in that** an indicator for indicating a mean blood pressure value is described in the two-dimensional region, the indicator being associated with the pulse wave pattern having the corresponding systolic blood pressure value and diastolic blood pressure value as peak values.

15. The bioinformation report according to claim 14, **characterized in that** the indicator for indicating the mean blood pressure value is a linear indicator.

16. The bioinformation report according to claim 14, **characterized in that** the indicator for indicating the mean blood pressure value is an indicator for visually indicating a region corresponding to blood pressure values equal to or lower than the mean blood pressure value of the pulse wave pattern or blood pressure values equal to or higher than the mean blood pressure value.

17. The bioinformation report according to any one of claims 12 to 16, **characterized in that** the pulse wave pattern is a pulse wave pattern measured almost at the same site as the systolic blood pressure value and the diastolic blood pressure value.

18. The bioinformation report according to any one of claims 12 to 15, **characterized in that** the pulse wave pattern is a pulse wave pattern measured almost at the same site as measurement of the blood pressure values, and
the plurality of pulse wave patterns are laid out in the two-dimensional region so as to have characteristic points on the same position in a horizontal axis direction of the two-dimensional coordinates.

19. The bioinformation report according to any one of claims 12 to 16, **characterized in that** the plurality of pulse wave patterns are presented so as to be visually different from one another.

# FIG. 1

24a ☐ ☐ ~24b 25a ◯ ◯ ~25b

EP 1 715 429 A1

```
                              203                    204                      205
   23              CARDIAC SOUND           ELECTROCARDIOGRAPH           PULSE WAVE
   ◯──             DETECTING UNIT          SIGNAL                       DETECTING UNIT
                                           DETECTING UNIT
```

```
  21R   21h      211R
  21aR                AVASCULARIZATION                        DISPLAYING UNIT      ~70
                      CONTROLLING
  21L                 UNIT FOR UPPER
  21aL                LIMBS
             21h                                              RECORDING UNIT       ~75
         211L      201
                                           OPERATION
                              10~          CONTROLLING
                                           UNIT
  22R   22h      221R   202                                   STORAGE UNIT         ~80
  22aR                AVASCULARIZATION
                      CONTROLLING
  22L                 UNIT FOR LOWER
  22aL                LIMBS                                   AUDIO OUTPUTTING UNIT  ~85
             22h
         221L

                                           INPUT INSTRUCTION   ~90
                                           UNIT
                                                                                   BODY
```

# F I G. 2

| | | |
|---|---|---|
| 1 | $= \dfrac{1}{k^2} \times \left( \ln \dfrac{Ps}{Pd} \right) \times (PWV)^2$ | $= \sqrt{\dfrac{1}{k^2} \times \left( \ln \dfrac{Ps}{Pd} \right) \times (PWV)^2}$ |
| 2 | $= k \times \dfrac{\left( \ln \dfrac{Ps}{Pd} \right) \times (PWV)^2}{(Ps - Pd)}$ | $= \sqrt{k \times \dfrac{\left( \ln \dfrac{Ps}{Pd} \right) \times (PWV)^2}{(Ps - Pd)}}$ |
| 3 | $= k \times \dfrac{(PWV)^2}{Pd}$ | $= \sqrt{k \times \dfrac{(PWV)^2}{Pd}}$ |
| 4 | $= \dfrac{1}{k^2} \times \left( \ln \dfrac{Pm}{Pd} \right) \times (PWV)^2$ | $= \sqrt{\dfrac{1}{k^2} \times \left( \ln \dfrac{Pm}{Pd} \right) \times (PWV)^2}$ |
| 5 | $= k \times \dfrac{\left( \ln \dfrac{Pm}{Pd} \right) \times (PWV)^2}{(Pm - Pd)}$ | $= \sqrt{k \times \dfrac{\left( \ln \dfrac{Pm}{Pd} \right) \times (PWV)^2}{(Pm - Pd)}}$ |

# FIG. 3

| BLOOD PRESSURE | RIGHT BRACHUM | LEFT BRACHUM | RIGHT ANKLE | LEFT ANKLE |
|---|---|---|---|---|
| SYS | 0 | 116 | 116 | 129 | 125 |
| DIA | 0 | 72 | 72 | 63 | 62 |
| MAP | | 85 | 85 | 81 | 87 |

← 310

EP 1 715 429 A1

14

# FIG. 4

EP 1 715 429 A1

# FIG. 5

|  | BLOOD PRESSURE | RIGHT BRACHUM | LEFT BRACHUM | RIGHT ANKLE | LEFT ANKLE |
|---|---|---|---|---|---|
| SYS | 0 | 140 | 140 | 135 | 134 |
| DIA | 0 | 82 | 82 | 78 | 75 |
| MAP |  | 107 | 107 | 98 | 104 |

← 310

# F I G. 6

| BLOOD PRESSURE | RIGHT BRACHUM | LEFT BRACHUM | RIGHT ANKLE | LEFT ANKLE | |
|---|---|---|---|---|---|
| SYS | 0 | 108 | 110 | 95 | 61 |
| DIA | 0 | 58 | 52 | 50 | 42 | ← 310 |
| MAP | | 90 | 73 | 71 | 50 |

[mmHg]

321 322 323 324
325 326 327 328

~320

EP 1 715 429 A1

**F I G. 7A**

**F I G. 7B**

# F I G. 8

| Examination Result | | | Date of Measurement : September 1, 2005 14:38:12 |
|---|---|---|---|

Name : Fukuda, Ichiro
ID : 00000001      Age : 48      Sex : Male

Height : 177 cm     Weight : 60 kg
BMI : 19.1kg/m²     Heart Rate : 59 beats/minute

| Hardness of Artery | Blood Vessel Elasticity | haPWV | Findings |
|---|---|---|---|
| Right | 7.1 | | Blood vessel hardness of late thirties |
| Left | 7.4 | | |

| Blood pressure | Right Brachum | 180/120(150) | Seriously high blood pressure |
|---|---|---|---|

| Blood Circulation of Extremities | Blood pressure barycenter (BPB) | Suspicion of hemodynamic disorder on right foot |
|---|---|---|
| | (164∞,16mmHg) | Suspicion of hemodynamic disorder on left foot |

/340

Blood Pressure [mmHg]

| Right Brachum | Left Brachum | Right Ankle | Left Ankle |
|---|---|---|---|
| 180/120 (150) | 160/105 (120) | 130/60 (100) | 125/55 (90) |

150
100
50

350

250

150

50

ECG × 4

PCG × 4

Right arm × 1/2

Left arm × 1/2

Right foot × 1
%MAP 38
UT 126

Left foot × 1/2
%MAP 39
UT 134

| L = L 1 + L 2 + L 3 [cm] | PEP | 100 | R-AI | 0.78 |
|---|---|---|---|---|
| 138 70 37 31 | ET | 285 | PEP/ET | 0.35 |

Second Examination Department, ... Hospital

# FIG. 9

```
┌─────────────────────────────────────┐
│           REPORT OUTPUT              │
└─────────────────────────────────────┘
                   │
                   ▼
┌─────────────────────────────────────┐
│     READ DATA CORRESPONDING         │ ～ S101
│       TO SELECTED SUBJECT           │
└─────────────────────────────────────┘
                   │
                   ▼
┌─────────────────────────────────────┐
│       LAY OUT EACH DATA IN          │ ～ S103
│      REPORT OUTPUT FORMAT           │
└─────────────────────────────────────┘
                   │
                   ▼
┌─────────────────────────────────────┐
│      CONVERT DATA TO FORMAT         │ ～ S105
│    SUITABLE FOR OUTPUT DEVICE       │
└─────────────────────────────────────┘
                   │
                   ▼
┌─────────────────────────────────────┐
│      OUTPUT TO OUTPUT DEVICE        │ ～ S107
└─────────────────────────────────────┘
                   │
                   ▼
            ┌─────────────┐
            │     END     │
            └─────────────┘
```

**European Patent Office**

**EUROPEAN SEARCH REPORT**

Application Number

EP 06 11 2847

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | EP 1 306 049 A (COLIN CORPORATION)<br>2 May 2003 (2003-05-02)<br><br><br>* figure 3 *<br>* abstract *<br>* paragraph [0006] - paragraph [0008] *<br>* sentence 47, paragraph 11 *<br>* sentence 52, paragraph 11 - sentence 55 *<br>* paragraph [0037] *<br>* sentence 31, paragraph 39 *<br>* paragraph [0045] - paragraph [0046] *<br>----- | 1,5-7,<br>11-13,<br>17-19<br>2-4,<br>14-16 | INV.<br>G06F17/00<br>A61B5/0285 |
| X<br><br>A | US 2004/171941 A1 (NARIMATSU KIYOYUKI ET AL) 2 September 2004 (2004-09-02)<br><br><br>* figures 1,2,6 *<br>* paragraph [0025] - paragraph [0027] *<br>* paragraph [0034] *<br>* paragraph [0041] *<br>* paragraph [0044] *<br>----- | 1,8-10<br><br>2-7,<br>11-19 | |
| A | US 2002/120199 A1 (OGURA TOSHIHIKO ET AL) 29 August 2002 (2002-08-29)<br>* figure 4 *<br>* figures 5-7 *<br>* abstract *<br>----- | 1-19 | TECHNICAL FIELDS<br>SEARCHED (IPC)<br><br>G06F<br>A61B |
| A | US 5 261 414 A (AUNG ET AL)<br>16 November 1993 (1993-11-16)<br>* figure 6 *<br>* abstract *<br>-----<br>-/-- | 1-19 | |

The present search report has been drawn up for all claims

| Place of search | Date of completion of the search | Examiner |
|---|---|---|
| The Hague | 11 July 2006 | Schwenke, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 06 11 2847

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | US 4 924 871 A (HONEYAGER ET AL) 15 May 1990 (1990-05-15) * abstract; figure 6 * ----- | 1-19 | |
| A | PATENT ABSTRACTS OF JAPAN vol. 016, no. 194 (C-0938), 11 May 1992 (1992-05-11) & JP 04 028346 A (NIPPON KODEN CORP), 30 January 1992 (1992-01-30) * abstract; figure 5 * ----- | 1-19 | |
| A | PATENT ABSTRACTS OF JAPAN vol. 2003, no. 12, 5 December 2003 (2003-12-05) & JP 2004 236679 A (NAKAMURA YOSHINOBU; NAKAMURA KIYOHARU), 26 August 2004 (2004-08-26) * abstract; figures 2,3 * ----- | 1-19 | |
| A | US 2003/187361 A1 (SU TSUNG-KUN) 2 October 2003 (2003-10-02) * abstract; figure 4 * ----- | 19 | TECHNICAL FIELDS SEARCHED (IPC) |

The present search report has been drawn up for all claims

| Place of search | Date of completion of the search | Examiner |
|---|---|---|
| The Hague | 11 July 2006 | Schwenke, S |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 06 11 2847

This annex lists the patent family members relating to the patent documents cited in the above-mentioned European search report.
The members are as contained in the European Patent Office EDP file on
The European Patent Office is in no way liable for these particulars which are merely given for the purpose of information.

11-07-2006

| Patent document cited in search report | | Publication date | Patent family member(s) | | Publication date |
|---|---|---|---|---|---|
| EP 1306049 | A | 02-05-2003 | CN | 1416776 A | 14-05-2003 |
| | | | JP | 2003126054 A | 07-05-2003 |
| | | | TW | 531404 B | 11-05-2003 |
| | | | US | 2003083580 A1 | 01-05-2003 |
| US 2004171941 | A1 | 02-09-2004 | JP | 3683257 B2 | 17-08-2005 |
| | | | JP | 2004261321 A | 24-09-2004 |
| US 2002120199 | A1 | 29-08-2002 | NONE | | |
| US 5261414 | A | 16-11-1993 | DE | 4220532 A1 | 14-01-1993 |
| | | | FR | 2678157 A1 | 31-12-1992 |
| | | | GB | 2257529 A | 13-01-1993 |
| | | | JP | 5003858 A | 14-01-1993 |
| US 4924871 | A | 15-05-1990 | JP | 2001223 A | 05-01-1990 |
| | | | JP | 2664982 B2 | 22-10-1997 |
| JP 04028346 | A | 30-01-1992 | JP | 1884805 C | 10-11-1994 |
| | | | JP | 6002125 B | 12-01-1994 |
| JP 2004236679 | A | 26-08-2004 | NONE | | |
| US 2003187361 | A1 | 02-10-2003 | DE | 10249264 A1 | 02-10-2003 |
| | | | JP | 2003290159 A | 14-10-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2000316821 A